# EUROPÄISCHE PATENTSCHRIFT

(11) **EP 4 000 587 B1**
(45) Veröffentlichungstag und Bekanntmachung des Hinweises auf die Patenterteilung: **03.01.2024**
(21) Anmeldenummer: 20209611.1
(22) Anmeldetag: 24.11.2020
(51) Int. Cl.: A61J 1/14, B65D 51/00, B65D 55/02

(54) **VERSCHLUSSSYSTEM FÜR EINEN MEDIKAMENTENBEHÄLTER SOWIE MEDIKAMENTENBEHÄLTER MIT EINEM VERSCHLUSSSYSTEM**
CLOSURE SYSTEM FOR A DRUG CONTAINER AND DRUG CONTAINER WITH A CLOSURE SYSTEM
SYSTÈME DE FERMETURE POUR UN RÉCIPIENT DE MÉDICAMENTS AINSI QUE RÉCIPIENT DE MÉDICAMENTS DOTÉ D'UN SYSTÈME DE FERMETURE

(43) Veröffentlichungstag der Anmeldung: 25.05.2022
(73) Patentinhaber: Trenta2 S.r.l., 39100 Bozen (IT)
(72) Erfinder: GALLMETZER, Dietrich, 39018 Terlan (IT)
(74) Vertreter: Tergau & Walkenhorst Intellectual Property GmbH

(56) Entgegenhaltungen:
- US-A1- 2004 141 886
- US-A1- 2006 091 098
- US-A1- 2009 306 620
- US-B1- 6 223 918
- US-B2- 9 731 878

## Beschreibung

Die Erfindung betrifft ein Verschlusssystem für einen Medikamentenbehälter, dessen Innenraum über eine in der Art einer Flaschenmündung ausgestaltete Behälteröffnung zugänglich ist. Sie bezieht sich weiter auf einen Medikamentenbehälter mit einem solchen Verschlusssystem sowie auf eine Verwendung des Verschlusssystems.

Medikamente, insbesondere für die Behandlung in hoch spezialisierten oder komplexen Therapien, werden üblicherweise in Wirkstoff- oder Medikamentenbehältern, auch als Container oder Phiole bezeichnet, bereitgestellt. Ein solcher Medikamentenbehälter ist üblicherweise in der Art eines Fläschchens ausgestaltet und umfasst einen Innenraum, in dem das Medikament oder der Wirkstoff vorgehalten wird und der über eine in der Art einer Flaschenmündung ausgestaltete Behälteröffnung zugänglich ist. Von einem solchen Behälter oder Container aus wird der Wirkstoff dann über geeignete Transfersysteme für die eigentliche Verabreichung an geeignete Systeme wie beispielsweise eine Spritze oder eine intravenöse Leitung, die einen Flüssigkeitszugang zum Kreislauf des Patienten bereitstellt, übergeben.

Gerade in modernen medizinischen Verfahren oder Therapien können Medikamente oder Substanzen zum Einsatz kommen, die an sich eigentlich toxisch oder auf sonstige Weise schädlich oder gefährlich sind. Für das mit der Handhabung solcher Substanzen betraute Personal, wie beispielsweise Apotheker und Krankenschwestern, können somit akute und langfristige Gesundheitsrisiken entstehen, gerade wenn es wiederholt Medikamenten oder Lösungsmitteln ausgesetzt ist, die während der Zubereitung, der Verabreichung von Medikamenten und anderen ähnlichen Behandlungen in die Luft entweichen könnten. Dieses Problem kann besonders schwerwiegend sein, wenn es sich um Zytotoxine, antivirale Medikamente, Antibiotika oder Radiopharmazeutika handelt. Die durch die Exposition gegenüber diesen Medikamenten potentiell entstehenden Gesundheitsrisiken umfassen ein erhöhtes Krebsrisiko, genetische Veränderungen und dergleichen.

Des Weiteren ist es auch im Hinblick auf den Umstand, dass in jüngster Zeit Medikamente mit einem äußerst hohen Dosispreis zugelassen worden sind, dringend wünschenswert oder sogar notwendig, die unbeabsichtigte Abgabe auch kleinster Mengen solcher Medikamente oder Wirkstoffe an die Umgebung zuverlässig zu vermeiden. Zu diesem Zweck sind so genannte geschlossene Transfersysteme gebräuchlich, bei denen in der Art einer gekapselten Ausführung in jeder Phase des Flüssigkeitstransfers sichergestellt ist, dass der Wirkstoff oder die von diesem freigesetzten Gase oder Aerosole nicht in die Umgebung entweichen können.

Für eine ordnungsgemäße Funktionsweise derartiger Systeme mit der angestrebten Vermeidung unbeabsichtigter Wirkstoffverluste ist aber auch die geeignete Ausgestaltung der Medikamentenbehälter notwendig. Üblicherweise sind die Wirkstoff- oder Medikamentenbehälter dazu mit geeigneten Verschlusssystemen versehen, bei denen ein Verschlussstopfen die Behälteröffnung verschließt. Dieser Verschlussstopfen kann dann für eine Entnahme des Medikaments beispielsweise mittels einer Hohlnadel durchstochen werden, über die das Medikament dann aus dem Behälter herausgesaugt werden kann. Zur Fixierung des Verschlussstopfens kann dabei eine Aufprellkappe mit einem eine zentrale Öffnung aufweisenden Ringdeckel vorgesehen sein, der an der "Flaschenmündung" mit der Behälteröffnung angebracht werden kann. Der Verschlussstopfen wird dann zentral in diesem Ringdeckel angebracht. Ein solches Verschlusssystem ist aus US2004/141886 bekannt.

Selbst bei derartigen Verschlusssystemen hat sich jedoch herausgestellt, dass nicht in allen Fällen die gewünschte Dichtigkeit des Systems und damit einhergehend eine möglichst weit gehende Vermeidung unerwünschter Freisetzungen der Medikamente oder Wirkstoffe gewährleistet werden kann.

Der Erfindung liegt daher die Aufgabe zugrunde, ein Verschlusssystem der oben genannten Art anzugeben, mit dem die Dichtigkeit des Systems insgesamt noch weiter verbessert werden kann. Des Weiteren sollen ein Medikamentenbehälter mit einem solchen Verschlusssystem sowie eine besonders günstige Verwendung eines solchen Verschlusssystems angegeben werden.

Bezüglich des Verschlusssystem für einen Medikamentenbehälter, dessen Innenraum über eine in der Art einer Flaschenmündung ausgestaltete Behälteröffnung zugänglich ist, wird diese Aufgabe erfindungsgemäß gelöst, indem das Verschlusssystem umfasst:
- eine Aufprellkappe mit einem eine zentrale Öffnung aufweisenden Ringdeckel, an dessen Außenumfang eine Anzahl von mit einer an der Behälteröffnung umlaufend angebrachten Außenwulst in Eingriff bringbaren Rastelementen angeordnet ist, und
- einen einstückigen Verschlussstopfen mit einer Verschlussplatte, an deren erster Plattenseite eine die zentrale Öffnung des Ringdeckels vollständig ausfüllende, mit dieser rastend in Eingriff bringbare Verdickung angeformt ist, und an deren zweiter Plattenseite ein Radial-Dichtelement mit einem an die lichte Weite der Behälteröffnung angepassten, im Hinblick auf die Verformbarkeit des Materials des Verschlussstopfens geringfügig größer als die lichte Weite der Behälteröffnung gewählten Querschnitt angeformt ist.

Vorteilhafte Ausgestaltungen der Erfindung sind Gegenstand der Unteransprüche.

Die Erfindung geht dabei von der Überlegung aus, dass für eine besonders hohe Dichtigkeit des Gesamtsystems und zur besonders weitgehenden Vermeidung unerwünschter Wirkstoffverluste die Dichtwirkung des Verschlussstopfens besonders umfassend ausgestaltet werden sollte. Wie sich völlig überraschend herausgestellt hat, sind hierbei mehrere Aspekte zur Auslegung des Verschlusstopfens zu berücksichtigen. In herkömmlichen Systemen ist der Verschlusstopfen, üblicherweise auf Basis eines gummiartigen oder hinreichend elastischen Materials, dafür ausgelegt, in die Behälteröffnung ein- und auf deren Begrenzungs- oder Mündungsrand aufgelegt zu werden. Durch ein zugeordnetes Befestigungselement, beispielsweise eine geeignete, auf den Mündungsrand aufrastbare Aufprellkappe, wird der Rand des Verschlussstopfens dann auf den Mündungsrand der Behälteröffnung aufgedrückt, so dass er infolge der Verformbarkeit des Materials seine Dichtwirkung entfalten kann. Ein solches System nutzt zur Dichtwirkung aber lediglich axiale Kraftkomponenten, also Kraftkomponenten in einer Richtung parallel zur Längs- oder Zentralachse der "Flaschenmündung" des Behälters.

Um die dabei erreichbare Dichtwirkung noch weiter zu erhöhen, sollten zusätzlich aber auch radiale Kraftkomponenten, also Anpresskräfte, die den Verschlusstopfen in radialer Richtung an die Innenseite der Behältermündung andrücken, nutzbar gemacht werden. Um dem Rechnung zu tragen, sollte der Verschlussstopfen in seinem in die Behälteröffnung hineinragenden Bereich hinsichtlich der Formgebung und Dimensionierung seines Querschnitts derart ausgestaltet sein, dass unter Berücksichtigung der Verformbarkeit seines Materials eine flächige Andrück- oder Anpresswirkung an die Innenwand der Behältermündung entsteht.

Vorteilhafterweise sind die Komponenten hinsichtlich ihrer Materialwahl in besonderem Maße an die jeweils vorgesehene Funktionalität angepasst: die Aufprellkappe sollte dabei für die beabsichtige Schnapp- oder Rastverbindung auf der Behälteröffnung bzw. einen diese umlaufenden Rastrand geeignet und insbesondere entsprechend steif ausgeführt sein. Demgegenüber sollte der Verschlusstopfen im Hinblick auf die gewünschte leichte Verformung bei der Herstellung der Dichtwirkung geeignet ausgeführt sein. Dementsprechend ist die Aufprellkappe vorzugsweise aus einem Kunststoff, besonders bevorzugt aus Polypropylen (PP), einem Polyolefin, Cyclo-Olefin-Copolymer (COC), Cyclo-Olefine-Polymer (COP) oder Polycarbonat, gefertigt. In alternativer oder zusätzlicher vorteilhafter Weiterbildung ist der Verschlussstopfen aus Gummi oder aus TPE gefertigt.

Für eine besonders sichere und zuverlässige Handhabung sollte das Verschlusssystem nach seiner Anbringung am Medikamentenbehälter besonders gesichert sein. Dazu umfassend es vorteilhafterweise als weitere Komponente einen auf die Aufprellkappe aufschiebbaren Sicherungsring, der mittels eines innenseitig angeformten, endseitig umlaufend angeordneten Schnapprandes rastend an der Aufprellkappe fixierbar ist. Nach der Anbringung der Aufprellkappe, bei der die an ihrem Außenumfang angeordneten Rastelemente beim Aufschieben auf die Behältermündung mit der an der Behälteröffnung umlaufend angebrachten Außenwulst in Eingriff gebracht, also "aufgeschnappt" oder eingerastet, werden, kann der Sicherungsring auf die Aufprellkappe aufgeschoben werden. Dabei umschließt er die Rastelemente ringförmig von außen und fixiert sie somit in der eingerasteten Stellung; ein Ausweichen nach außen und ein damit verbundenes Lösen der Verrastung ist dann nicht mehr möglich.

In ganz besonders bevorzugter und als eigenständig erfinderisch angesehener Ausgestaltung ist an der Aufprellkappe, besonders bevorzugt an deren Ringdeckel, ein RFID-Chip angeordnet. Dieser kann mit einer Codierung oder Kennung versehen sein, die den Medikamentenbehälter individuell kennzeichnet und/oder weitere Informationen bezüglich des Inhalts, beispielsweise der Medikamenten- oder Wirkstoffzusammensetzung, ein eventuelles Verfallsdatum oder dergleichen enthalten kann. Insbesondere da ein solcher Chip auch kontaktlos ausgelesen werden kann, kann eine automatisierte Handhabung des Medikamentenbehälters und seines Inhalts, bis hin zu einer voll oder teilweise automatisierten Medikamentenherstellung in Mischsystemen oder dergleichen, erreicht werden.

In einer als eigenständig erfinderisch angesehenen Ausgestaltung ist ein Verschlusssystem der oben genannten Art, vorzugsweise in Verbindung mit der vorstehend erläuterten Ausgestaltung, mit einem Originalitätsverschluss für den Medikamentenbehälter versehen. Das Verschlusssystem kann dabei mit einem zusätzlich äußeren Verschlusselement in der Art eines Einwegverschlusses versehen sein. Dieser Einwegverschluss, der beispielsweise einen abreissbaren oder verplombt ausgeführten Siegeldeckel umfassen kann, erlaubt eine problemlose und zuverlässige Identifikation, ob der Container bereits zum Flüssigkeitstransfer verwendet wurde oder nicht, und erleichtert somit die Zuordnung, ob der Container bereits "angebrochen" ist und somit bevorzugt für eine weitere Flüssigkeitsentnahme verwendet werden sollte, bis er vollständig entleert ist und somit entsorgt werden sollte.

In dieser als eigenständig erfinderisch angesehenen Ausführungsform des Verschlusssystems für einen Medikamentenbehälter, dessen Innenraum über eine in der Art einer Flaschenmündung ausgestaltete Behälteröffnung zugänglich ist, umfasst dieses:
- eine Aufprellkappe mit einem eine zentrale Öffnung aufweisenden Ringdeckel, an dessen Außenumfang eine Anzahl von mit einer an der Behälteröffnung umlaufend angebrachten Außenwulst in Eingriff bringbaren Rastelementen angeordnet ist,
- einen einstückigen Verschlussstopfen mit einer Verschlussplatte, an deren erster Plattenseite eine die zentrale Öffnung des Ringdeckels vollständig ausfüllende, mit dieser rastend in Eingriff bringbare Verdickung angeformt ist, und
- einen außenseitig fest an der Aufprellkappe angebrachten Originalitätsverschluss mit einem Abreißring, an den in seinem Zentralbereich eine die zentrale Öffnung des Ringdeckels vollständig überdeckende Siegelplatte angeformt ist.

Der Originalitätsverschluss umfasst dabei vorteilhafterweise zur festen Verbindung mit der Aufprellkappe ein zwischen dem Abreißring und der Siegelplatte verlaufendes, über eine Anzahl von Sollbruchstellen an den Abreißring und/oder an die Siegelplatte angeformtes Verbindungssegment.

Bezüglich des Medikamentenbehälters, dessen Innenraum über eine in der Art einer Flaschenmündung ausgestaltete Behälteröffnung zugänglich ist, wird die genannte Aufgabe gelöst, indem er mit einem Verschlusssystem der vorstehend beschriebenen Art versehen ist.

Ebenfalls als eigenständig erfinderisch angesehen wird die Verwendung eines Verschlusssystems der genannten Art für einen Medikamentenbehälter.

Die mit der Erfindung erzielten Vorteile bestehen insbesondere darin, dass durch die geeignete Ausgestaltung des Verschlussstopfens gezielt Dichtflächen in radialer Richtung, also zur Innenwand des den Verschlussstopfens umgebenden Bereichs der Flaschenmündung hin, bereitgestellt werden. Diese können einen zusätzlichen Dichteffekt bewirken und damit die Dichtigkeit des Systems insgesamt verbessern.

Ein Ausführungsbeispiel der Erfindung wird anhand einer Zeichnung näher erläutert. Darin zeigen:
- Fig. 1: einen mit einem Verschlusssystem verschlossenen Medikamentenbehälter,
- Fig. 2: den Medikamentenbehälter nach Fig. 1 im Längsschnitt in Explosionsdarstellung,
- Fig. 3: den Medikamentenbehälter nach Fig. 1 im Längsschnitt,
- Fig. 4: einen Verschlussstopfen für den Medikamentenbehälter nach Fig. 1,
- Fig. 5: eine Aufprellkappe des Verschlusssystems in Draufsicht,
- Fig. 6: einen Originalitätsverschluss des Verschlusssystems in verschiedenen Ansichten,
- Fig. 7: eine Sequenz von Schritten bei der Anbringung des Verschlusssystems an den Medikamentenbehälter nach Fig. 1,
- Fig. 8: den verschlossenen Medikamentenbehälter 1,
- Fig. 9: den Medikamentenbehälter 1 gem. Fig. 8, nachdem der Originalitätsverschluss entfernt wurde,
- Fig. 10: den Medikamentenbehälter 1 gem. Fig. 8 im angeschlossenen Zustand,
- Fig. 11: einen mit einem alternativen Verschlusssystem verschlossenen Medikamentenbehälter,
- Fig. 12: den Verschlussstopfen des alternativen Verschlusssystems nach Fig. 11,
- Fig. 13: die Aufprellkappe des alternativen Verschlusssystems nach Fig. 11,
- Fig. 14: den mit dem alternativen Verschlusssystem versehenen Medikamentenbehälter im Längsschnitt,
- Fig. 15: eine alternative Ausführungsform eines Originalitätsverschlusses für den Medikamentenbehälter nach Fig. 11, und
- Fig. 16: eine Sequenz von Schritten bei der Anbringung des alternativen Verschlusssystems an den Medikamentenbehälter nach Fig. 11.

Gleiche Teile sind in allen Figuren mit denselben Bezugszeichen versehen.

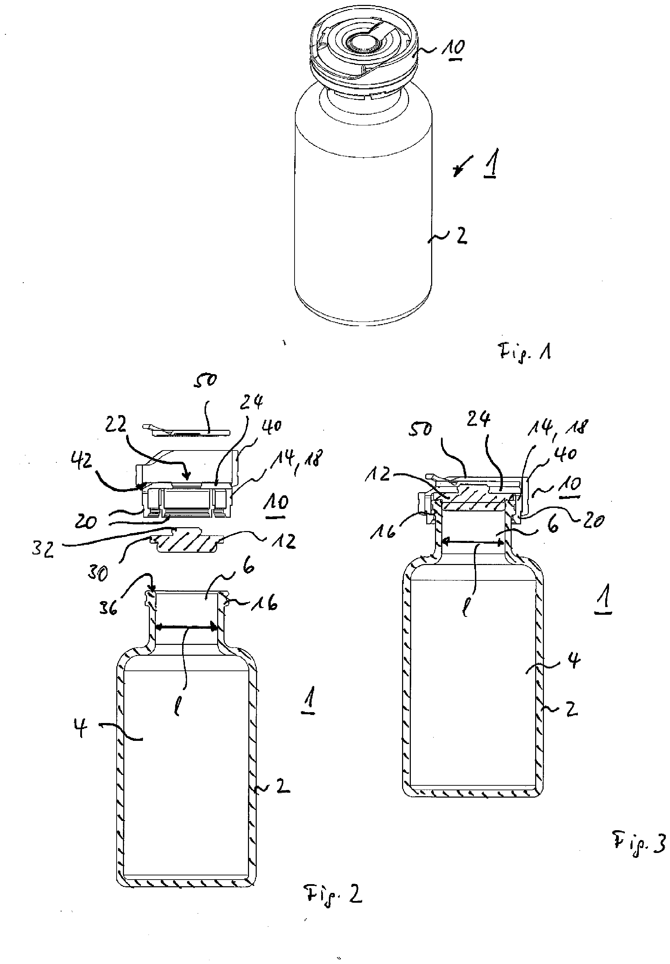

Der Medikamentenbehälter 1 gem. Fig. 1, auch als Container oder Phiole bezeichnet, ist in der Art eines Fläschchens ausgestaltet. Er umfasst einen von einer Behälterwand 2 umschlossenen Innenraum 4, in dem das Medikament oder der Wirkstoff vorgehalten wird. Die Behälterwand 2 ist im Ausführungsbeispiel aus Glas ausgeführt, kann aber auch aus einem anderen geeigneten, insbesondere für die sichere Verwahrung sensibler Stoffe geeigneten, Material wie beispielsweise einem geeignet gewählten Kunststoff mit oder ohne Barriereschicht gefertigt sein. Besonders bevorzugt ist hierbei ein "medical grade" Kunststoff vorgesehen wie beispielsweise COP Varianten 690R^{®}, 790R^{®}, COC Varianten Topas^{®} 8007S-04, 6013S-04, 6015S-04. Der Innenraum 4 ist über eine in der Art einer Flaschenmündung ausgestaltete Behälteröffnung 6 zugänglich.

Der Medikamentenbehälter 1 ist in besonderem Maße für die Bereithaltung von Wirkstoffen oder Medikamenten ausgestaltet, bei denen jegliche Materialverluste durch unbeabsichtigte Freisetzung an die Umwelt oder Umgebung möglichst weitgehend vermieden werden sollen. Dies kann beispielsweise der Fall sein für toxische, gesundheitsgefährdende oder anderweitig für die handhabenden Personen gefährliche Substanzen, oder auch für besonders hochpreisige Substanzen oder Wirkstoffe, wie sie insbesondere in modernen Therapien vermehrt zum Einsatz kommen. Um solche unerwünschten Materialverluste besonders gering zu halten, ist der Medikamentenbehälter 1 mit einem die Behälteröffnung 6 verschließenden Verschlusssystem 10 ausgerüstet, das für eine besonders hohe Dichtigkeit ausgelegt ist.

Das Verschlusssystem 10 ist seinerseits mehrkomponentig ausgeführt und umfasst als wesentliche Komponenten einen Verschlussstopfen 12 zum Verschließen der Behälteröffnung 6 und eine Fixierkappe 14, mit der der Verschlussstopfen 12 fest an der Behälteröffnung 6 angebracht werden kann.

Im Ausführungsbeispiel ist die Behälterwand 2 des Medikamentenbehälters 1 im Bereich der Behälteröffnung 6 mit einer umlaufend angebrachten Außenwulst 16 als Befestigungselement versehen. Daran angepasst ist die Fixierkappe 14 als Aufprellkappe 18 ausgeführt, an deren Außenumfang eine Anzahl von mit der Außenwulst 16 in Eingriff bringbaren Schnapphaken oder Rastelementen 20 angeordnet ist. Beim Anbringen der Aufprellkappe 18 kann diese somit auf die Behälteröffnung 6 aufgesteckt oder aufgeprellt werden, wobei die Rastelemente 20 zunächst durch die Außenwulst 16 nach außen gebogen werden und anschließend, nach weiterem Aufschieben, die Außenwulst hintergreifen und in der Art einer Schnappverbindung mit dieser verrasten. Ein solches Aufprellsystem ist besonders einfach montierbar und damit bevorzugt; alternativ könnte aber auch eine Schraubverbindung der Fixierkappe 14 mit der Behälterwand 2 oder eine andere geeignete Verbindung vorgesehen sein.

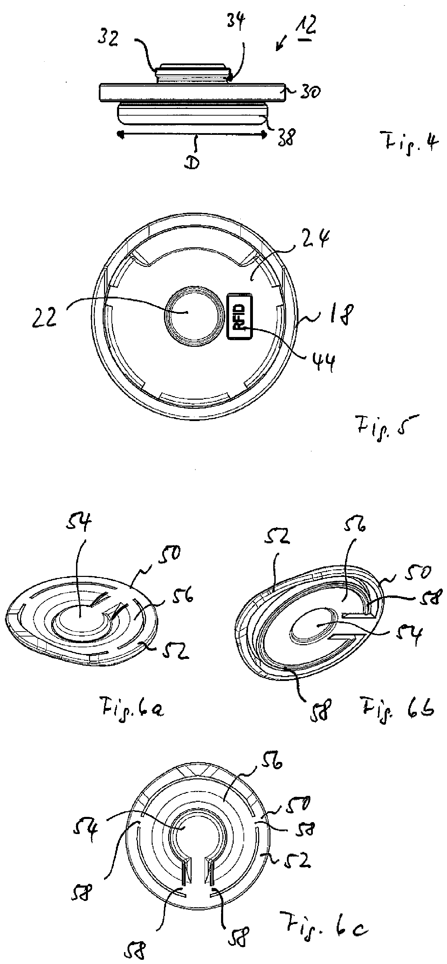

Die Aufprellkappe 18 umfasst einen eine zentrale Öffnung 22 aufweisenden Ringdeckel 24. Der an sich einstückig ausgeführte Verschlussstopfen 12 umfasst hingegen in der Art eines Grundelements eine Verschlussplatte 30, an deren erster Plattenseite eine die zentrale Öffnung 22 des Ringdeckels 24 vollständig ausfüllende, mit dieser rastend in Eingriff bringbare Verdickung 32 angeformt ist, wie dies besonders deutlich in der vergrößerten Darstellung des Verschlussstopfens 12 in seitlicher Ansicht in Fig. 4 erkennbar ist. Die Verdickung 32 ist in ihrem Verbindungsbereich mit der Verschlussplatte 30 mit einer eine Hinterschneidung bildenden umlaufenden Nut 34 versehen. Der an sich einstückige Verschlussstopfen 12 ist, auch im Hinblick auf die erwünschten Dichtzwecke, aus einem geeigneten und zudem vergleichsweise weichen und gut verformbaren Material, im Ausführungsbeispiel aus Gummi oder aus TPE, bevorzugt "medical grade", gefertigt. Durch diese Materialwahl ist auch sichergestellt, dass der Verschlussstopfen 12 bei Bedarf, also wenn Wirkstoff aus dem Medikamentenbehälter 1 entnommen werden soll, mittels eines geeigneten Instruments, beispielsweise einer Hohlnadel, durchstochen werden kann. Unter Nutzung dieser Materialeigenschaften, insbesondere der Verformbarkeit, kann der Verschlussstopfen 12 mit der Aufprellkappe 18 annähernd fest verbunden werden, indem die Verdickung 32 in die Öffnung 22 im Ringdeckel 24 eingebracht wird und der umlaufende Rand der Öffnung 22 anschließend in die Nut 34 eingreift und damit den Verschlussstopfen 12 an der Aufprellkappe 18 fixiert.

Zur vorliegend besonders erwünschten Abdichtung der Behälteröffnung 6 trägt der Verschlussstopfen 12 auf zweierlei Weise bei. Einerseits wird, mit bekannten System durchaus vergleichbar, eine Dichtwirkung erzielt, indem im montierten System (wie dies im Längsschnitt in Fig. 3 gezeigt ist) die in ihren Abmessungen, insbesondere ihrem Außendurchmesser, geeignet an den Mündungsrand 36 der Behälteröffnung 6 angepasste Verschlussplatte 30 durch die auf den Mündungsrand 36 aufrastbare Aufprellkappe 18 mit ihrem Rand des Verschlussstopfens auf den Mündungsrand 36 aufgedrückt wird. Durch diese in Bezug auf die Längsachse der Behälteröffnung gesehen axiale Kraftwirkung kann die Verschlussplatte 30 infolge der Verformbarkeit des Materials bereits eine Dichtwirkung entfalten kann. Darüber hinaus ist vorliegend für eine insgesamt besonders erhöhte Dichtwirkung aber auch noch die Bereitstellung von radialen Kraftkomponenten, also Anpresskräfte, die den Verschlusstopfen 12 in radialer Richtung an die Innenseite der Behälterwand 2 im Bereich ihrer Mündung andrücken, vorgesehen.

Dazu ist, wie dies ebenfalls aus der vergrößerten Darstellung in Fig. 4 deutlich wird, an der zweiter Plattenseite der Verschlussplatte 30 ein Radial-Dichtelement 38 angeformt. Das Radial-Dichtelement 38 ist in seiner Querschnittsform an die Querschnittsform der Behälteröffnung 6 im Mündungsbereich angepasst (im Ausführungsbeispiel sind beide rund). Hinsichtlich seiner Dimensionierung ist es zudem an die lichte Weite I der Behälteröffnung 6 angepasst und im Hinblick auf die Verformbarkeit des Materials des Verschlussstopfens 12 geringfügig größer als die lichte Weite I der Behälteröffnung 6 ausgeführt. Damit entsteht bei in die Behälteröffnung 6 eingebrachtem Radial-Dichtelement 38 unter Berücksichtigung der Verformbarkeit seines Materials eine flächige Andrück- oder Anpresswirkung an die Innenwand des Behälters im Bereich der Behälteröffnung. Im Hinblick auf gängige Standards und übliche Normen für derartige Komponenten kann die Behälteröffnung geeignet gewählt und dimensioniert sein; beispielsweise kann ihre lichte Weite geeignet abgestimmt auf das Standard-Maß "13er Neck" (entspricht einem Außendurchmesser der Behälteröffnung von 13mm) oder auf das Standard-Maß "20er Neck" (entspricht einem Außendurchmesser der Behälteröffnung von 20mm) gewählt sein.

Die Aufprellkappe 18 besteht im Ausführungsbeispiel aus einem geeignet gewählten Kunststoff, nämlich aus Polypropylen (PP), einem Polyolefin, Cyclo-Olefin-Copolymer (COC), Cyclo-Olefine-Polymer (COP) oder Polycarbonat.

Als weitere Komponente, wie dies wiederum aus der Darstellung in Fig. 2 deutlich wird, umfasst das Verschlusssystem 10 einen auf die Aufprellkappe 18 aufschiebbaren Sicherungsring 40. Dieser kann nach dem Aufprellen der Aufprellkappe 18 und der Verrastung mit der Außenwulst 16 von außen umgreifend auf die Aufprellkappe 18 geschoben werden. Damit fixiert er die Rastelemente 20 radial, so dass diese nicht mehr nach außen ausweichen können. Damit ist die Verrastung der Aufprellkappe 18 mit der Außenwulst 16 nicht mehr ohne weiteres lösbar und somit festgelegt. Der Sicherungsring 40 weist seinerseits einen innenseitig angeformten, endseitig umlaufend angeordneten Schnapprand 42 auf, mit dem er rastend an der Aufprellkappe 18 fixierbar ist.

Wie der Darstellung in Fig. 5 der Aufprellkappe 18 in Draufsicht gut entnehmbar ist, ist an ihrem Ringdeckel 24 ein RFID-Chip 44 angeordnet. Dieser kann mit einer Codierung oder Kennung versehen sein, die den Medikamentenbehälter individuell kennzeichnet und/oder weitere Informationen bezüglich des Inhalts, beispielsweise der Medikamenten- oder Wirkstoffzusammensetzung, ein eventuelles Verfallsdatum oder dergleichen enthalten kann. Insbesondere da ein solcher Chip auch kontaktlos ausgelesen werden kann, kann eine automatisierte Handhabung des Medikamentenbehälters 1 und seines Inhalts, bis hin zu einer voll oder teilweise automatisierten Medikamentenherstellung in Mischsystemen oder dergleichen, erreicht werden.

In weiterer, als eigenständig erfinderisch angesehener Ausgestaltung weist das Verschlusssystem 10, wie beispielsweise Fig. 2 gut entnehmbar ist, als Komponente einen Originalitätsverschluss 50 auf. Dieser soll in der Art eines Einwegverschlusses sicherstellen, dass für den Verwender auf einfache und zuverlässige festgestellt werden kann, ob der Medikamentenbehälter 1 bereits zum Flüssigkeitstransfer verwendet wurde oder nicht, d. h. ob bereits Wirkstoff entnommen wurde oder nicht. Er erleichtert somit die Zuordnung, ob der Container bereits "angebrochen" ist und somit bevorzugt für eine weitere Flüssigkeitsentnahme verwendet werden sollte, bis er vollständig entleert ist und somit entsorgt werden sollte. Der Originalitätsverschluss 50 ist in Fig. 6 im Detail in verschiedenen Ansichten gezeigt, nämlich in seitlicher Ansicht von oben (Fig. 6a), in seitlicher Ansicht von unten (Fig. 6b) und in Draufsicht (Fig. 6c). dabei einen außenseitig fest an der Aufprellkappe 18 angebracht. In Anlehnung an Originalitätsverschlüsse aus dem Getränkebereich ist der Originalitätsverschluss 50 mit einem Abreißring 52 versehen, an den im Zentralbereich des Originalitätsverschlusses 50 eine Siegelplatte 54 angeformt ist. Die Siegelplatte 54 ist dabei derart dimensioniert und positioniert, dass sie im montierten Zustand die zentrale Öffnung 22 des Ringdeckels 24 und damit die hierüber zugängliche frei liegende Fläche des Verschlussstopfens 12 vollständig überdeckt. Für einen Zugriff auf das Innere des Medikamentenbehälters 1, also für eine Entnahme von Wirkstoff, muss somit zunächst die Siegelplatte 54 entfernt werden, so dass der Verschlussstopfen 12 durchstochen werden kann.

Der Originalitätsverschluss 50 weist zur festen Verbindung mit der Aufprellkappe 18 ein Verbindungssegment 56 auf. Dieses ist segmentweise um die Siegelplatte 54 umlaufend zwischen dem Abreißring 52 und der Siegelplatte 54 angeordnet und über eine Anzahl von Sollbruchstellen 58 an den Abreißring 52 und an die Siegelplatte 54 angeformt. Durch diese Bauweise ist sichergestellt, dass zunächst der Originalitätsverschluss 50 als Ganzes fest mit der Aufprellkappe 18 verbunden werden kann. Bei der Betätigung des Abreissrings 52, also zum Entfernen der Siegelplatte 54, werden sodann die Sollbruchstellen 58 durchtrennt, so dass sich das Ensemble aus Abreissring 52 und Siegelplatte 54 entfernen lässt. Da das Verbindungssegment 56 unverändert an seiner Position auf der Aufprellkappe 18 verbleibt, ist sodann leicht erkennbar, dass das Siegel gebrochen und bereits Wirkstoff aus dem Medikamentenbehälter 1 entnommen wurde.

Zur festen Verbindung des Originalitätsverschlusses 50 mit der Aufprellkappe 18 ist an der Unterseite des Verbindungssegments 56 eine Schweiß-Verbindungsraupe 58 angeformt, wie dies der Darstellung in Fig. 6b entnehmbar ist. Die Schweißverbindungsraupe 58 ist dabei zur Herstellung der Verbindung mit der Oberseite der Aufprellkappe 18 durch einen Schweißvorgang, beispielsweise durch Ultraschallschweißen vorgesehen und entsprechend ausgelegt. Durch Auflegen auf die Aufprellkappe 18, ggf. angemessenes Andrücken und anschließendes Beaufschlagen mit einem Ultraschall-Schweißprozess kann dann das Verbindungssegment 56 und mit diesem der Originalitätsverschluss 50 insgesamt mit der Aufprellkappe 18 stoffschlüssig verbunden werden.

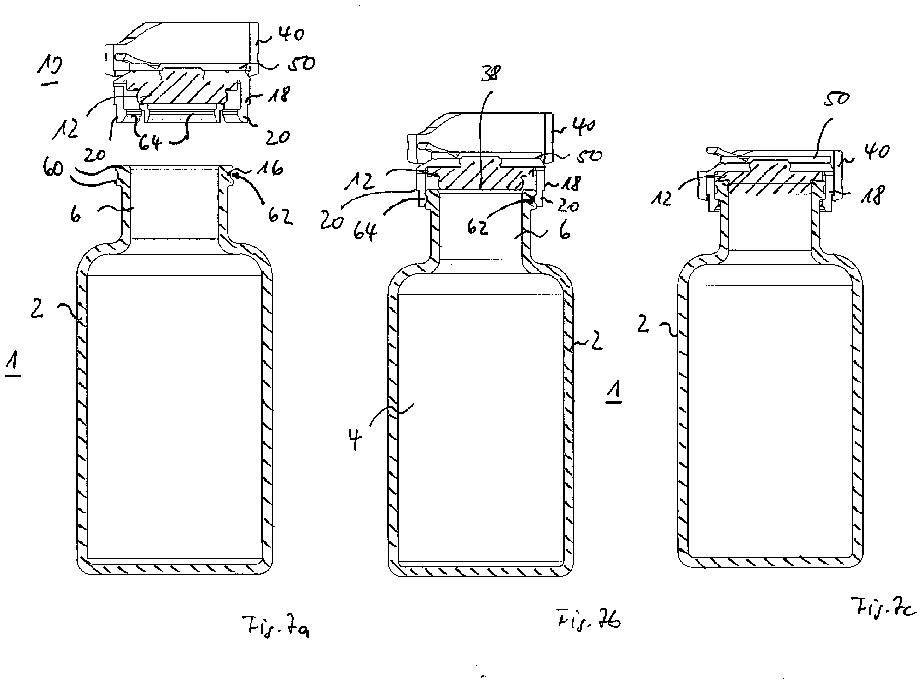

Die Anbringung des Verschlusssystems 10 an den Medikamentenbehälter 1 ist in Fig. 7 anhand einer Sequenz von Schritten gezeigt. In einem ersten Schritt, in Fig. 7a gezeigt, wird zunächst in als eigenständig erfinderisch angesehener Abfolge das Verschlusssystem 10 vormontiert. Dazu wird der Originalitätsverschluss 50, wie vorstehend beschrieben, mit der Aufprellkappe 18 fest verbunden, insbesondere verschweißt, und die Verdickung 32 des Verschlussstopfens 12 wird, wie ebenfalls vorstehend beschrieben, mit der Öffnung 22 des Ringdeckels 24 der Aufprellkappe 18 in Rastverbindung gebracht.

Ausgehend von diesem vormontierten Zustand kann unter Umständen und je nach Bedarf der in Fig. 7b gezeigte Zwischenschritt durchgeführt werden. Bei diesem Zwischenschritt wird die im Ausführungsbeispiel vorgesehene Ausführungsform genutzt, bei der die die Behälteröffnung 6 im Mündungsbereich außenseitig umlaufende Außenwulst 16 als Doppelwulst ausgeführt ist. Bei dieser Ausführung umfasst die Außenwulst 16 zwei parallel zueinander verlaufende Einzelwülste 60, die durch eine dazwischen verlaufende Nut 62 voneinander getrennt sind. In einer derartigen Bauweise ist es ermöglicht, das Verschlusssystem 10 in einem ersten Schritt lediglich derart weit auf die Behälteröffnung 6 aufzuschieben, dass die an die Rastelemente 20 jeweils angeformten Rasthaken 64 lediglich in die Nut 62 eingreifen und das System dort zunächst arretieren. In diesem Zwischenzustand, der in Fig. 7b gezeigt ist, verschließt der Verschlussstopfen 12 noch nicht die Behälteröffnung 6, da das Radial-Dichtelement 38 noch nicht in das Innere der Behälteröffnung 6 eingedrungen ist. Vielmehr wird in diesem Zustand ein umlaufender noch offener Ringspalt zwischen dem Radial-Dichtelement 38 und der Mündung des Behälters 1 gebildet. Gemeinsam mit den umfangsseitigen Zwischenräumen zwischen den einzelnen Rastelementen 20 der Aufprellkappe 18 besteht somit in diesem Zustand noch eine gasseitige Verbindung des Innenraums 4 des Medikamentenbehälters 1 und der äußeren Umgebung.

Diese Position kann beispielsweis zu einer Gefriertrocknung, auch als Lyophilisierung, Lyophilisation oder Sublimationstrocknung bezeichnet, des Wirkstoffs im Medikamentenbehälter 1 verwendet werden. Hierbei handelt es sich um ein mittlerweile weit verbreitetes Verfahren zur schonenden Trocknung von Produkten, das bei einer Vielzahl von Medikamenten oder Wirkstoffen eingesetzt wird, um diese haltbar zu machen. Bei einer solchen Gefriertrocknung kann es erforderlich sein, entstehende Gase oder Dämpfe, insbesondere Wasserdampf, an die Umgebung abgeben zu können, und eine solche Möglichkeit bietet die in Fig. 7b gezeigte Positionierung der Komponenten.

Nach diesem Zwischenschritt, oder eventuell auch direkt nach der in Fig. 7a gezeigten Vormontage, falls kein solcher Zwischenschritt benötigt wird, wird der Vorgang des Verschließens dann beendet und das System in den in Fig. 7c gezeigten, vollständig verschlossenen Zustand überführt. Dazu wird zunächst die Aufprellkappe 18 weiter über die Behälteröffnung bewegt, so dass der Verschlussstopfen 12 nunmehr mit seinem Radial-Dichtelement 38 in die Behälteröffnung 6 eindringt, bis die Verschlussplatte 30 mit ihrem äußeren Rand auf der Mündung des Behälters 1 aufliegt und anschließend, unter geringfügiger Verformung der Verschlussplatte 30 in Längsrichtung der Behälteröffnung 6 gesehen, die Rasthaken 64 der Aufprellkappe 18 unterhalb der zweiten oder unteren Einzelwulst 60 einrasten. Anschließend wird dann der Sicherungsring 40 nach unten hin verschoben, so dass er die Aufprellkappe 18 außenseitig umgreift. Damit werden die Rastelemente 20 in ihrer Position verriegelt, und der Medikamentenbehälter 1 in der in Fig. 7c gezeigten Position ist sicher verschlossen.

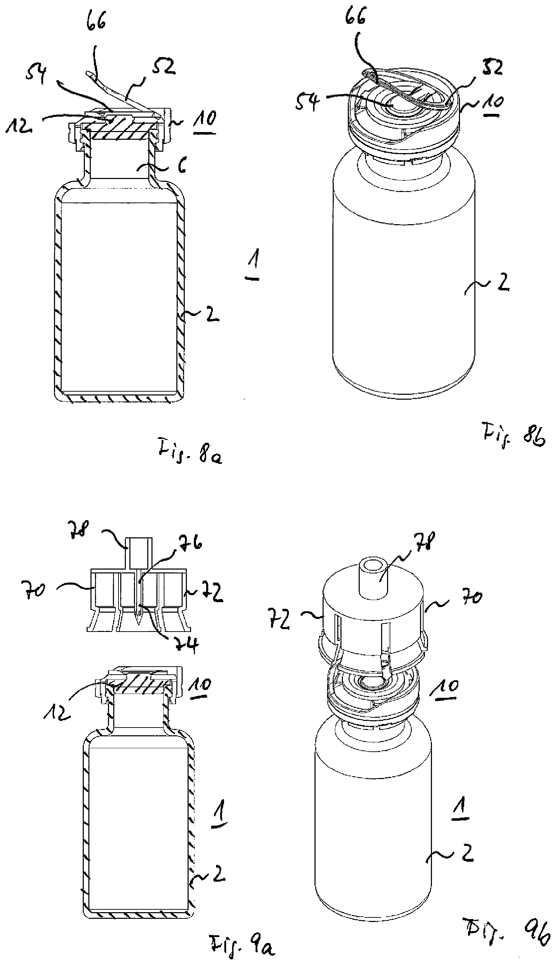

Zum Öffnen des solchermaßen verschlossenen Medikamentenbehälters 1 wird, wie dies in Fig. 8a im Längsschnitt und in Fig. 8b in perspektivischer Ansicht gezeigt ist, zunächst der Abreissring 52 des Originalitätsverschlusses 50 mittels eines integrierten, leicht ausgewölbten Betätigungssegments 66 leicht angehoben, wobei die den Abreissring 52 mit dem Verbindungssegment 56 verbindenden Sollbruchstellen 58 durchtrennt werden. Der Abreissring 52 bietet in dieser Position eine Eingriffsmöglichkeit, beispielsweise für den Finger des Verwenders, so dass er anschließend, ähnlich dem Verschluss einer Getränkedose, leicht vollständig entfernt werden kann. Dabei werden auch die die Siegelplatte 54 mit dem Verbindungssegment 56 verbindenden Sollbruchstellen 58 durchtrennt, und die Siegelplatte 54 kann vollständig gemeinsam mit dem Abreissring 52 von der Aufprellkappe 18 abgehoben werden. Damit wird der in der Öffnung 22 befindliche Teil des Verschlussstopfens 12 freigelegt und damit zugänglich. Gleichermaßen ist dadurch aber auch sichergestellt ist, dass das erfolgte Öffnen leicht identifizierbar ist und der Verwender somit erkennen kann, dass bereits ein Zugriff auf den Wirkstoff oder das Medikament erfolgt ist.

Nachdem somit das Siegel des Medikamentenbehälters 1 entfernt wurde und der Verschlussstopfen 12 für ein Durchstechen bereit gemacht wurde, kann die Verbindung des "entsicherten" Medikamentenbehälters 1 mit einem geeigneten Transfer- oder Entnahmesystem erfolgen. Dies ist beispielhaft in Fig. 9a im Längsschnitt und in Fig. 9b in perspektivischer Ansicht gezeigt. Das dort gezeigte Entnahmeelement 70 umfasst eine Gehäusekappe 72, die in ihrer Innenwand geeignet konturiert und konfiguriert ist, so dass sie auf das Verschlusssystem 10, insbesondere den Sicherungsring 40, aufgerastet werden kann. Innerhalb der Gehäusekappe 72 ist als eigentliches Entnahmeelement eine Hohlnadel 74 angeordnet, mittels derer der Verschlussstopfen 12 durchstochen werden kann. Der Innenkanal 76 ist medienseitig mit einem außenseitig der Gehäusekappe 72 angeordneten Anschlussstutzen 78 verbunden, an den beispielsweise ein Schlauch oder dergleichen angeordnet werden kann.

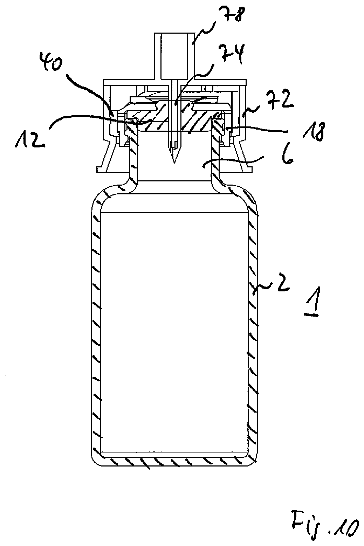

Ein auf diese Weise an ein Transfersystem oder dergleichen angeschlossener Medikamentenbehälter 1 ist mit durchstochenem Verschlussstopfen 12 in Fig. 10 gezeigt.

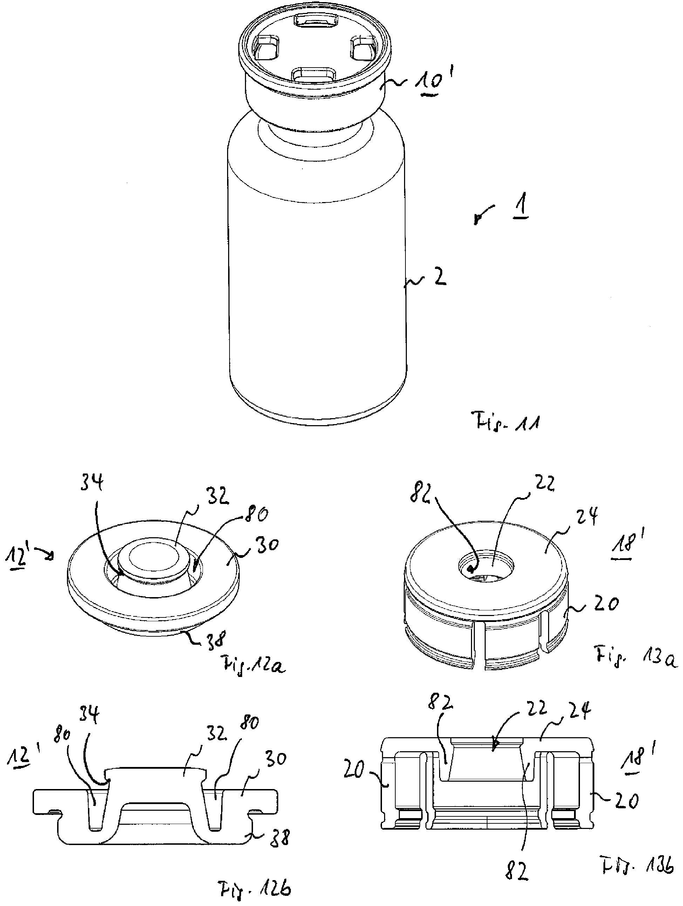

Ein mit einem alternativen Verschlusssystem 10' ausgerüsteter Medikamentenbehälter 1 ist in den Figs. 11 ff gezeigt. Das vom Grundkonzept her vergleichbar mit dem Verschlusssystem 10 aufgebaute alternative Verschlusssystem 10' unterscheidet sich vom ersteren im Wesentlichen durch die genaue Ausführung des Verschlussstopfens 12' und durch die genaue Ausführung des Originalitätsverschlusses 50'. Alle diese Komponenten werden als eigenständig erfinderisch angesehen und können erfindungsgemäß beliebig miteinander kombiniert vorgesehen sein.

Der Verschlussstopfen 12' in der alternativen Ausführungsform des Verschlusssystems 10' ist in Fig. 12 in perspektivischer Ansicht (Fig. 12a) und im Längsschnitt (Fig. 12b) gezeigt. Auch in dieser Ausführungsform umfasst er in der Art eines Grundelements die Verschlussplatte 30, an deren erster Plattenseite die die zentrale Öffnung 22 des Ringdeckels 24 vollständig ausfüllende, mit dieser rastend in Eingriff bringbare Verdickung 32 angeformt ist. Ebenso wie beim Verschlusssystem 10 ist auch in dieser Ausführungsform die Verdickung 32 in ihrem Verbindungsbereich mit der Verschlussplatte 30 mit einer eine Hinterschneidung bildenden umlaufenden Nut 34 versehen, und der an sich einstückige Verschlussstopfen 12' ist aus einem geeigneten und zudem vergleichsweise weichen und gut verformbaren Material, im Ausführungsbeispiel aus Gummi oder aus TPE gefertigt.

Der Verschlussstopfen 12' ist als eigenständig erfinderisch angesehener Ausgestaltung für eine noch weiter verbesserte Dichtwirkung in radialer Richtung ausgeführt. Dazu ist die Formgebung derart gewählt, dass der die Verdickung 32 ausbildende Zentralbereich des Verschlussstopfens 12' von einer tief in die Verschlussplatte 30 hineinreichenden, umlaufenden nut- oder grabenartigen Vertiefung 80 umgeben ist. Daran angepasst weist die Aufprellkappe 18' in dieser Ausführungsform, wie sie in Fig. 13a in perspektivischer Ansicht und in Fig. 13b im Längsschnitt dargestellt ist, einen an den Ringdeckel 24 an seiner Unterseite angeformten, die Öffnung 22 umlaufenden Verstärkungsring 82 auf. Bei der Montage der beiden Komponenten wird dieser Verstärkungsring 82 in die Vertiefung 80 des Verschlussstopfens 12' eingebracht. Die Abmessungen sind dabei derart aufeinander abgestimmt, dass der Verstärkungsring 82 dem Radial-Dichtelement 38 des Verschlussstopfens 12' erhöhte Festigkeit und Steifigkeit nach außen hin, also in radialer Richtung, verleiht und damit die radiale Abdichtung noch weiter verbessert. Insbesondere kann durch die entsprechend gewählten Abmessungen der Verstärkungsring 82 das Radial-Dichtelement 38 mehr oder weniger nach außen hin leicht verformen und dabei eine zusätzliche Anpresskraft in radialer Richtung an die Innenwand 4 des Medikamentenbehälters 1 im Bereich der Behälteröffnung 6 erzeugen.

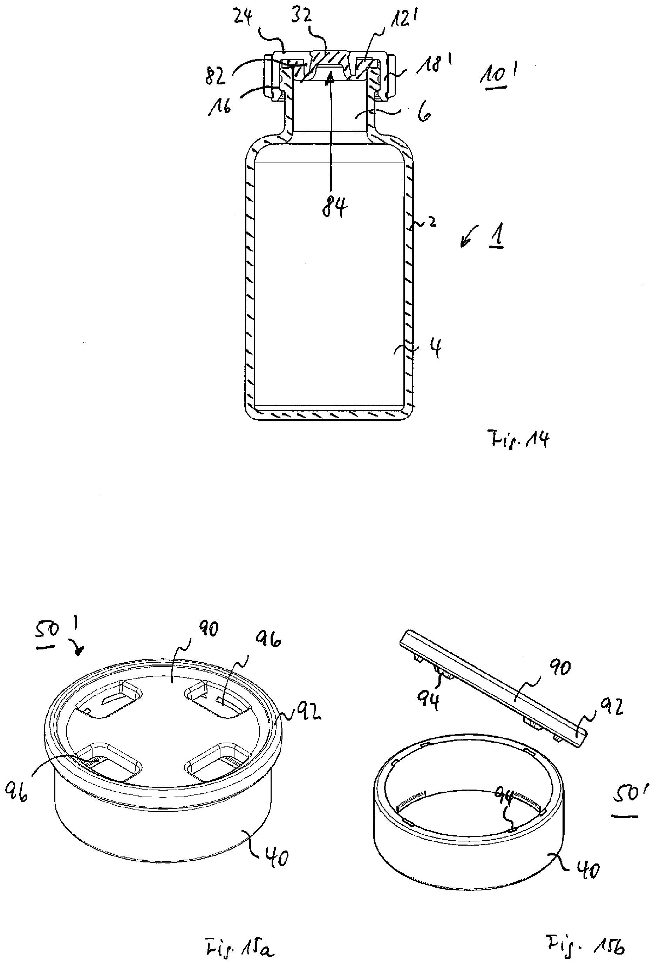

Diese Funktionsweise wird auch in der Darstellung des Medikamentenbehälters 1 mit Verschlusssystem 10' im Längsschnitt in Fig. 14 noch einmal deutlich. Dabei ist gut erkennbar wie der Verstärkungsring 82 in die Vertiefung 80 eingreift.

Des Weiteren ist dieser Darstellung entnehmbar, dass der Verschlussstopfen 12' direkt unterhalb der Verdickung 32 in seinem Zentralbereich auf seiner dem Innenraum 4 des Behälters 1 zugewandten Seite eine Ausnehmung 84 aufweist. Damit kann, ohne Beeinträchtigung der Dichtwirkung insgesamt, die Stärke des Verschlussstopfens 12' im Bereich unterhalb der Verdickung 32 vergleichsweise gering gehalten werden, so dass das Durchstechen mit einer Hohlnadel oder dergleichen auf besonders einfache Weise möglich ist.

Des Weiteren ist in ebenfalls als eigenständig erfinderisch angesehener Ausgestaltung auch der Originalitätsverschluss 50' in dieser Ausführungsform alternativ ausgestaltet, wie er in Fig. 15 in verschiedenen Ansichten gezeigt ist. Der Originalitätsverschluss 50' umfasst eine Deckelplatte 90, die über eine Anzahl von an ihrem wulstartig erweiterten umlaufenden Rand 92 über eine Anzahl von als Sollbruchstellen ausgebildeten Verbindungsstegen 94 fest mit dem auf die Aufprellkappe 18' aufschiebbaren Sicherungsring 40 verbunden ist. Die Deckelplatte 90, die in ihrem äußeren Randbereich mit einer Anzahl von Entlüftungsöffnungen 96 versehen ist, ist dabei in ihrem Zentralbereich flächig durchgehend ausgeführt, so dass sie im montierten Zustand die darunterliegende Öffnung 22 der Aufprellkappe 18' und damit die Verdickung 32 des Verschlussstopfens 12' vollständig bedeckt und damit sichert. Zum Öffnen des solchermaßen verschlossenen Medikamentenbehälters 1 wird die Deckelplatte 90 durch Anheben des umlaufenden Randes 92 angehoben, wobei die als Sollbruchstellen ausgebildeten Verbindungsstege 94 abgerissen oder durchtrennt werden. Damit wird auch bei diesem System der in der Öffnung 22 befindliche Teil des Verschlussstopfens 12' freigelegt und damit zugänglich.

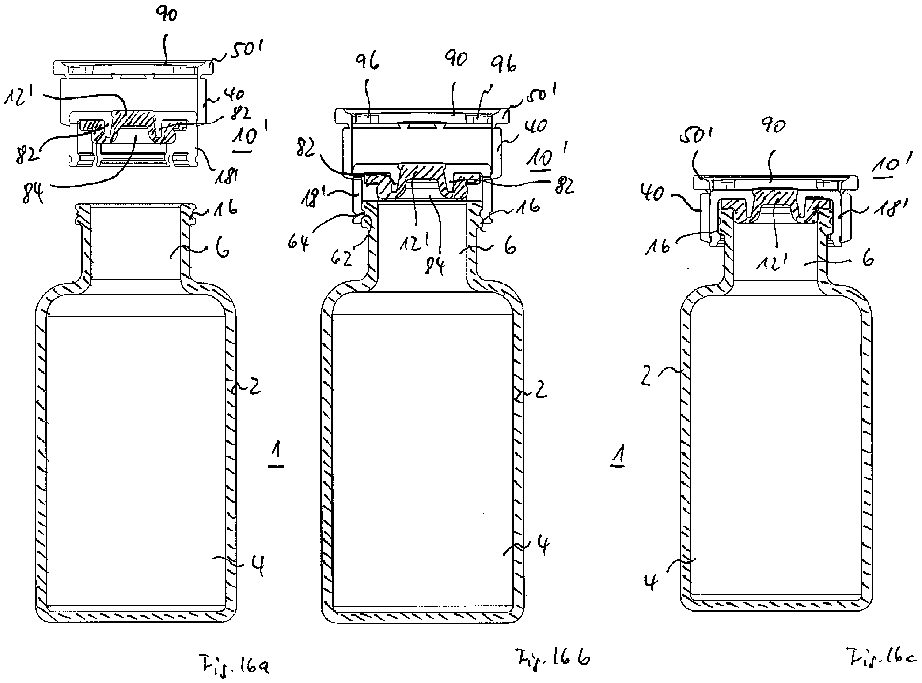

Die Anbringung des Verschlusssystems 10' an den Medikamentenbehälter 1 ist in Fig. 16 in Analogie zur Darstellung nach Fig. 7 anhand einer Sequenz von Schritten gezeigt. In einem ersten Schritt, in Fig. 16a gezeigt, wird auch hier zunächst in als eigenständig erfinderisch angesehener Abfolge das Verschlusssystem 10' vormontiert. Dazu wird die Verdickung 32 des Verschlussstopfens 12' zunächst mit der Öffnung 22 des Ringdeckels 24 der Aufprellkappe 18' in Rastverbindung gebracht.

Ausgehend von diesem vormontierten Zustand kann auch hier unter Umständen und je nach Bedarf, beispielsweise zur Durchführung einer Gefriertrocknung oder Lyophilisation, der in Fig. 16b gezeigte Zwischenschritt durchgeführt werden. Bei diesem Zwischenschritt wird die im Ausführungsbeispiel vorgesehene Ausführungsform genutzt, bei der die die Behälteröffnung 6 im Mündungsbereich außenseitig umlaufende Außenwulst 16 als Doppelwulst ausgeführt ist. Auch in dieser Ausführungsform kann das Verschlusssystem 10' bei Bedarf in einem ersten Schritt lediglich derart weit auf die Behälteröffnung 6 aufgeschoben werden, dass die an die Rastelemente 20 jeweils angeformten Rasthaken 64 lediglich in die Nut 62 eingreifen und das System dort zunächst arretieren. In diesem Zwischenzustand, der in Fig. 16b gezeigt ist, verschließt der Verschlussstopfen 12' noch nicht die Behälteröffnung 6, da das Radial-Dichtelement 38 noch nicht in das Innere der Behälteröffnung 6 eingedrungen ist. Vielmehr wird in diesem Zustand ein umlaufender noch offener Ringspalt zwischen dem Radial-Dichtelement 38 und der Mündung des Behälters 1 gebildet. Gemeinsam mit den umfangsseitigen Zwischenräumen zwischen den einzelnen Rastelementen 20 der Aufprellkappe 18' besteht somit in diesem Zustand noch eine gasseitige Verbindung des Innenraums 4 des Medikamentenbehälters 1 und der äußeren Umgebung.

Nach diesem Zwischenschritt, oder eventuell auch direkt nach der in Fig. 16a gezeigten Vormontage, falls kein solcher Zwischenschritt benötigt wird, wird der Vorgang des Verschließens dann beendet und das System in den in Fig. 16c gezeigten, vollständig verschlossenen Zustand überführt. Dazu wird zunächst die Aufprellkappe 18' weiter über die Behälteröffnung bewegt, so dass der Verschlussstopfen 12' nunmehr mit seinem Radial-Dichtelement 38 in die Behälteröffnung 6 eindringt, bis die Verschlussplatte 30 mit ihrem äußeren Rand auf der Mündung des Behälters 1 aufliegt und anschließend, unter geringfügiger Verformung der Verschlussplatte 30 in Längsrichtung der Behälteröffnung 6 gesehen, die Rasthaken 64 der Aufprellkappe 18' unterhalb der zweiten oder unteren Einzelwulst 60 einrasten. Anschließend wird dann der Sicherungsring 40 nach unten hin verschoben, so dass er die Aufprellkappe 18' außenseitig umgreift. Damit werden die Rastelemente 20 in ihrer Position verriegelt, und der Medikamentenbehälter 1 in der in Fig. 16c gezeigten Position ist sicher verschlossen.

### Bezugszeichenliste

- 1: Medikamentenbehälter
- 2: Behälterwand
- 4: Innenraum
- 6: Behälteröffnung
- 10,10': Verschlusssystem
- 12,12': Verschlussstopfen
- 14: Fixierkappe
- 16: Außenwulst
- 18,18': Aufprellkappe
- 20: Rastelemente
- 22: Öffnung
- 24: Ringdeckel
- 30: Verschlussplatte
- 32: Verdickung
- 34: Nut
- 36: Mündungsrand
- 38: Radial-Dichtelement
- 40: Sicherungsring
- 42: Schnapprand
- 50,50': Originalitätsverschluss
- 52: Abreissring
- 54: Siegelplatte
- 56: Verbindungssegment
- 58: Sollbruchstelle
- 60: Einzelwulst
- 62: Nut
- 64: Rasthaken
- 66: Betätigungssegment
- 70: Entnahmeelement
- 72: Gehäusekappe
- 74: Hohlnadel
- 76: Innenkanal
- 78: Anschlussstutzen
- 80: Vertiefung
- 82: Verstärkungsring
- 84: Ausnehmung
- 90: Deckelplatte
- 92: Rand

- D: Durchmesser
- L: lichte Weite

## Patentansprüche

1. Verschlusssystem (10, 10') für einen Medikamentenbehälter (1), dessen Innenraum (4) über eine in der Art einer Flaschenmündung ausgestaltete Behälteröffnung (6) zugänglich ist, wobei das Verschlusssystem (10, 10') umfasst:
- eine Aufprellkappe (18,18') mit einem eine zentrale Öffnung (22) aufweisenden Ringdeckel (24), an dessen Außenumfang eine Anzahl von mit einer an der Behälteröffnung (6) umlaufend angebrachten Außenwulst (16) in Eingriff bringbaren Rastelementen (20) angeordnet ist, und
- einen einstückigen Verschlussstopfen (12, 12') mit einer Verschlussplatte (30), an deren erster Plattenseite eine Verdickung (32) angeformt ist, und an deren zweiter Plattenseite ein Radial-Dichtelement (38) mit einem an die lichte Weite (I) der Behälteröffnung (6) angepassten, im Hinblick auf die Verformbarkeit des Materials des Verschlussstopfens (12, 12') geringfügig größer als die lichte Weite (I) der Behälteröffnung (6) gewählten Querschnitt angeformt ist, **dadurch gekennzeichnet, dass** die Verdickung die zentrale Öffnung (22) des Ringdeckels (24) vollständig ausfüllt und mit dieser rastend in Eingriff bringbar ist.

2. Verschlusssystem (10, 10') nach Anspruch 1, dessen Aufprellkappe (18, 18') aus einem Kunststoff, vorzugsweise aus Polypropylen (PP), einem Polyolefin, Cyclo-Olefin-Copolymer (COC), Cyclo-Olefine-Polymer (COP) oder Polycarbonat gefertigt ist.

3. Verschlusssystem (10, 10') nach Anspruch 1 oder 2, dessen Verschlussstopfen (12, 12') aus Gummi oder aus TPE gefertigt ist.

4. Verschlusssystem (10, 10') nach einem der Ansprüche 1 bis 3, ferner umfassend einen auf die Aufprellkappe (18, 18') aufschiebbaren Sicherungsring (40), der mittels eines innenseitig angeformten, endseitig umlaufend angeordneten Schnapprandes (42) rastend an der Aufprellkappe (18, 18') fixierbar ist.

5. Verschlusssystem (10, 10') nach einem der Ansprüche 1 bis 4, bei dem am Ringdeckel (24) der Aufprellkappe (18, 18') ein RFID-Chip (44) angeordnet ist.

6. Verschlusssystem (10, 10') nach einem der Ansprüche 1 bis 5, mit einem einstückigen Verschlussstopfen (12, 12') mit einer Verschlussplatte (30), an deren erster Plattenseite eine die zentrale Öffnung (22) des Ringdeckels (24) vollständig ausfüllende, mit dieser rastend in Eingriff bringbare Verdickung (32) angeformt ist, und
- einen außenseitig fest an der Aufprellkappe (18, 18') angebrachten Originalitätsverschluss (50, 50') mit einem Abreißring (52), an den in seinem Zentralbereich eine die zentrale Öffnung (22) des Ringdeckels (24) vollständig überdeckende Siegelplatte (54) angeformt ist.

7. Verschlusssystem (10, 10') nach Anspruch 6, dessen Originalitätsverschluss (50, 50') zur festen Verbindung mit der Aufprellkappe (18, 18') ein zwischen dem Abreißring (32) und der Siegelplatte (54) verlaufendes, über eine Anzahl von Sollbruchstellen (58) an den Abreißring (52) und/oder an die Siegelplatte (54) angeformtes Verbindungssegment (56) umfasst.

8. Medikamentenbehälter (1), dessen Innenraum (4) über eine in der Art einer Flaschenmündung ausgestaltete Behälteröffnung (6) zugänglich ist, die mit einem Verschlusssystem (10, 10') nach einem der Ansprüche 1 bis 7 versehen ist.

9. Verwendung eines Verschlusssystems (10, 10') nach einem der Ansprüche 1 bis 7 für einen Medikamentenbehälter (1).

## Claims

1. A closure system (10, 10') for a medicinal product container (1) the interior space (4) of which is accessible via a container opening (6) configured in the manner of a bottle mouth (6), wherein the closure system (10, 10') comprises:
- a snap-on cap (18, 18') with an annular cover (24) having a central opening (22), on the outer circumference of which a plurality of snap-fitting elements (20) are disposed which can be brought into engagement with a continuous outer bead (16) mounted on the container opening (6), and
- a one-piece closure stopper (12, 12') with a closure plate (30) with an enlargement (32) formed on its first side, and a radial sealing element (38) is formed on its second side with a cross section which is selected to match the internal width (1) of the container opening (6) and which is slightly larger than the internal width (1) of the container opening (6) in view of the deformability of the material of the closure stopper (12, 12'), **characterized in that** the enlargement completely fills the central opening (22) of the annular cover (24) and can be snapped into engagement therewith.

2. The closure system (10, 10') as claimed in claim 1, the snap-on cap (18, 18') of which is manufactured from a plastic, preferably from polypropylene (PP), a polyolefin, cyclo-olefin copolymer (COC), cyclo-olefin polymer (COP) or polycarbonate.

3. The closure system (10, 10') as claimed in claim 1 or claim 2, the closure stopper (12, 12') of which is manufactured from rubber or from TPE.

4. The closure system (10, 10') as claimed in one of claims 1 to 3, further comprising a securing ring (40) which can be pushed onto the snap-on cap (18, 18'), which can be secured on the snap-on cap (18, 18') by snap-fitting by means of an internally formed continuous snap rim (42) disposed on the end.

5. The closure system (10, 10') as claimed in one of claims 1 to 4, in which a RFID chip (44) is disposed on the annular cover (24) of the snap-on cap (18, 18').

6. The closure system (10, 10') as claimed in one of claims 1 to 5, with a one-piece closure stopper (12, 12') with a closure plate (30) with an enlargement (32) formed on its first side which completely fills the central opening (22) of the annular cover (24) and which can be snapped into engagement therewith, and
- a tear-off closure (50, 50') securely attached to the outside of the snap-on cap (18, 18'), with a tear-off ring (52) on which a closure plate (54) is formed in its central region which completely covers the central opening (22) of the annular cover (24).

7. The closure system (10, 10') as claimed in claim 6, wherein for a secure connection to the snap-on cap (18, 18'), its tear-off closure (50, 50') comprises a connecting segment (56) which runs between the tear-off ring (52) and the closure plate (54), formed on the tear-off ring (52) and/or on the closure plate (54) via a plurality of predetermined breaking points (58).

8. A medicinal product container (1) the interior space (4) of which is accessible via a container opening (6) which is configured in the manner of a bottle mouth, which is provided with a closure system (10, 10') as claimed in one of claims 1 to 7.

9. Use of a closure system (10, 10') as claimed in one of claims 1 to 7 as a medicinal product container (1).

## Revendications

1. Système de fermeture (10, 10') pour un récipient de médicaments (1) dont l'intérieur (4) est accessible par une ouverture de récipient (6) formée à la manière d'un goulot de bouteille, dans lequel le système de fermeture (10, 10') comprend :
- un capuchon encliquetable (18, 18') comprenant un couvercle annulaire (24) présentant une ouverture centrale (22), sur le pourtour extérieur duquel est disposé un nombre d'éléments d'encliquetage (20) pouvant être mis en prise avec un bourrelet extérieur (16) posé sur tout le pourtour de l'ouverture de récipient (6), et
- un bouchon de fermeture (12, 12') d'une seule pièce comprenant une plaque de fermeture (30) sur le premier côté de laquelle est formé un épaississement (32), et sur le second côté de laquelle est formé un élément d'étanchéité radial (38) avec une section adaptée au diamètre intérieur (1) de l'ouverture de récipient (6), choisie légèrement plus grande que le diamètre intérieur (1) de l'ouverture de récipient (6) au vu de la déformabilité du matériau du bouchon de fermeture (12, 12'), **caractérisé en ce que** l'épaississement remplit complètement l'ouverture centrale (22) du couvercle annulaire (24) et peut être mis en prise par encliquetage avec celle-ci.

2. Système de fermeture (10, 10') selon la revendication 1, dont le capuchon encliquetable (18, 18') est fabriqué en plastique, de préférence en polypropylène (PP), polyoléfine, copolymère de cyclo-oléfine (COC), polymère de cyclo-oléfine (COP) ou polycarbonate.

3. Système de fermeture (10, 10') selon la revendication 1 ou 2, dont le bouchon de fermeture (12, 12') est fabriqué en caoutchouc ou en TPE.

4. Système de fermeture (10, 10') selon l'une des revendications 1 à 3, comprenant en outre une bague de sécurité (40) pouvant être poussée sur le capuchon encliquetable (18, 18') qui peut être fixée par encliquetage sur le capuchon encliquetable (18, 18') au moyen d'un bord encliquetable (42) disposé en extrémité sur le pourtour et formé à l'intérieur.

5. Système de fermeture (10, 10') selon l'une des revendications 1 à 4, dans lequel une puce RFID (44) est disposée sur le couvercle annulaire (24) du capuchon encliquetable (18, 18').

6. Système de fermeture (10, 10') selon l'une des revendications 1 à 5, comprenant un bouchon de fermeture (12, 12') d'une seule pièce comprenant une plaque de fermeture (30) sur le premier côté de laquelle est formé un épaississement (32) remplissant complètement l'ouverture centrale (22) du couvercle annulaire (24), pouvant être mis en prise par encliquetage avec celle-ci,
- une fermeture inviolable (50, 50') posée fixement sur le côté extérieur du capuchon encliquetable (18, 18'), comprenant une bague détachable (52) sur laquelle, en sa partie centrale, est formée une plaque d'étanchéité (54) recouvrant complètement l'ouverture centrale (22) du couvercle annulaire (24).

7. Système de fermeture (10, 10') selon la revendication 6, dont la fermeture inviolable (50, 50'), pour la liaison fixe avec le capuchon encliquetable (18, 18'), comprend un segment de liaison (56) passant entre la bague détachable (52) et la plaque d'étanchéité (54), formée sur la bague annulaire (52) et/ou sur la plaque d'étanchéité (54) par un nombre de points de rupture de consigne (58).

8. Récipient de médicaments (1) dont l'intérieur (4) est accessible par une ouverture de récipient (6) formée à la manière d'un goulot de bouteille qui est doté d'un système de fermeture (10, 10') selon l'une des revendications 1 à 7.

9. Utilisation d'un système de fermeture (10, 10') selon l'une des revendications 1 à 7 pour un récipient de médicaments (1).
